(19) Europäisches Patentamt
European Patent Office
Office européen des brevets

(11) Publication number: **0 062 544 B1**

(12) **EUROPEAN PATENT SPECIFICATION**

(45) Date of publication of patent specification: **30.09.87**

(51) Int. Cl.$^4$: **C 07 D 307/88**, B 41 M 5/12

(21) Application number: **82301885.8**

(22) Date of filing: **08.04.82**

(54) **New phthalide derivatives, process for preparing the same and recording system utilizing the same as colourless chromogenic material.**

(30) Priority: **08.04.81 JP 53678/81**
**13.03.82 JP 39965/82**

(43) Date of publication of application:
**13.10.82 Bulletin 82/41**

(45) Publication of the grant of the patent:
**30.09.87 Bulletin 87/40**

(84) Designated Contracting States:
**CH DE FR GB LI**

(56) References cited:
**US-A-4 020 056**
**US-A-4 022 771**
**US-A-4 107 428**

**The file contains technical information submitted after the application was filed and not included in this specification**

(73) Proprietor: **KANZAKI PAPER MANUFACTURING COMPANY LIMITED**
**9-8, Ginza 4-chome Chuo-ku**
**Tokyo 104 (JP)**

(72) Inventor: **Mitsuru, Kondo**
**1-11 Motomachi Jyokoji**
**Amagaski Hyogo (JP)**
Inventor: **Tomoyuki, Okimoto**
**1-11 Motomachi Jyokoji**
**Amagaski Hyogo (JP)**
Inventor: **Nobuo, Kanda**
**1-11 Motomachi Jyokoji**
**Amagaski Hyogo (JP)**

(74) Representative: **Seaborn, George Stephen et al**
**c/o Edward Evans & Co. Chancery House**
**53-64 Chancery Lane**
**London WC2A 1SD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

There are known various kinds of record systems utilizing the colourforming reaction between a colourless chromogenic material and an electron accepting acidic reactant material by means of mechanical, heat, electric or light energy. Among them are included a pressure-sensitive recording sheet, a heat-sensitive record sheet, an electrothermal recording sheet, an ultrasonic record sheet, an electron beam recording sheet, an electrostatic record sheet and a photosensitive recording sheet. The colourless chromogenic materials of these kinds also find their usefulness in typewriter ribbons, ball-point pen ink, crayon and stamp ink.

One of the most typical colourless chromogenic materials is crystal violet lactone. This dye material reacts with an electron accepting acidic reactant material upon contact to develop a clear colour of bluish violet but the developed colour has a poor light resistance so that the recording images (colour images) soon disappear in a short time when subjected to radiation of ultraviolet rays of daylight. Another disadvantage of this type of dye is in the fact that the recorded images obtained with this material show no absorption for the infrared range of 700—900 nm and accordingly this type of dye material cannot be used for a reading machine utilizing an optical reading responsive to infrared absorption.

US—A—4,020,056 discloses phthalide derivatives useful as chromogenic materials for producing heat- and pressure-sensitive recording materials. The phthalide derivatives according to the present invention differ from their prior art mainly in that they comprise at least two ring amino functions.

US—A—4,022,771 discloses phthalide derivatives useful as chromogenic materials for producing heat- and pressure-sensitive recording materials. The developed colour images obtained with the use of the phthalide derivatives disclosed in this U.S. patent have an absorption at 600 to 800 nm.

US—A—4,107,428 disclose phthalide derivatives useful as chromogenic materials for producing heat- and pressure-sensitive recording materials. The phthalide derivatives disclosed by this U.S. patent are different from the phthalide derivatives according to the present invention since compounds comprised by this document are disclaimed from this patent.

Our divisional application, EP—A—0 127 203, discloses phthalide derivatives which are also useful as chromogenic materials for producing heat- and pressure-sensitive recording materials.

The invention provides: novel phthalide derivatives useful as colourless chromogenic materials for use in various recording systems;

— novel colourless chromogenic materials for use in recording systems in which the colour images when developed therefrom have a good light resistance, especially a good ultraviolet ray resistance;

— novel colourless chromogenic materials for use in recording systems in which the colour images when developed therefrom show a good absorption for infrared rays;

— a novel process for preparing the aforementioned phthalide derivatives; and

an improved recording system in which a new phthalide derivative is used as a colourless chromogenic material and the colour images when developed therefrom have a good light resistance and show a good absorption for infrared rays in the range of 700—900 nm.

Summary of the Invention

The novel phthalide derivatives according to the invention have the general formula (I)

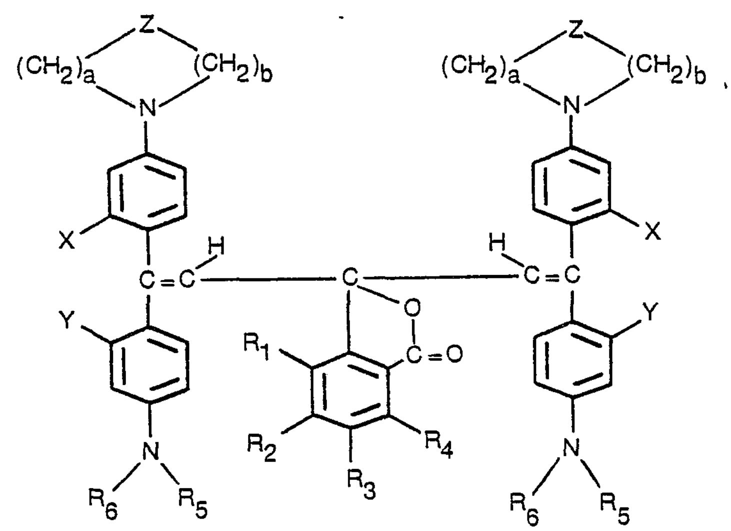

I

wherein:

each of $R_1$, $R_2$, $R_3$ and $R_4$ is hydrogen, halogen, $C_{1-4}$alkyl, $C_{1-4}$alkoxy, nitro $C_{1-4}$alkyl substituted amino or pyrrolidino;

each of $R_6$ and $R_5$ adjacent a common nitrogen atom is hydrogen, $C_{1-4}$alkyl, benzyl, phenyl or $C_{1-4}$alkyl substituted phenyl or $R_6$ and $R_5$ together with the adjacent nitrogen atom form a pyrrolidino, piperidino, hexamethylenimino or morpholino ring, both $R_6$'s being the same as each other and both $R_5$'s being the same as each other;

X is hydrogen, $C_{1-4}$alkyl or $C_{1-4}$alkoxy, both X's being the same as each other;

Y is hydrogen, $C_{1-4}$alkyl or $C_{1-4}$alkoxy, both Y's being the same as each other;

each Z is oxygen or methylene, both Z's being the same as each other;

each of a and b is an integer of from 1 to 3, both a's being the same as each other and both b's being the same as each other; and

in each N-heterocyclic ring $a + b \geqslant 3$;

provided that when all of $R_1$, $R_2$, $R_3$ and $R_4$ are chlorine or bromine X and/or Y is $C_{1-4}$alkyl or $C_{1-4}$alkoxy.

The phthalide derivatives having the above general formula (I) can be used as colourless chromogenic materials for use in various recording systems including a pressure sensitive recording system and a heat sensitive recording system. The compounds according to the invention can produce a colour of blue to green upon contact with an electron accepting acidic reactant material. The colour images produced have a good light resistance and can maintain their clear colour tone initially produced for a long time. The colour images produced also show a good absorption for infrared rays with the range of 700—900 nm so that they can be detected for reading in an infrared ray responsive optical reading machine.

A specific group of compounds according to the invention are those of the general formula: (II) comprised by formula (I)

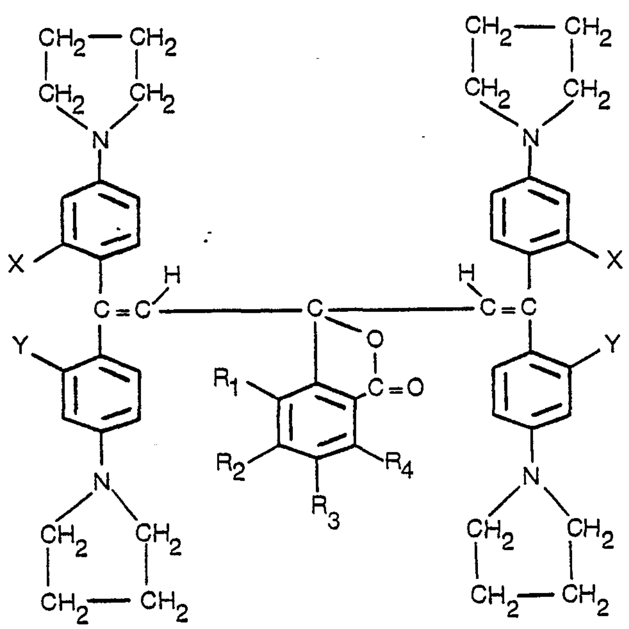

II

where $R_1$, $R_2$, $R_3$, $R_4$ and X and Y are defined above and X and Y are the same.

Compounds of the general formula (I) may be prepared by reacting together phthalic anhydride derivatives of the general formula (III) given below and ethylene derivatives of the general formula (IV) also given below. The reaction may be represented by the equation:

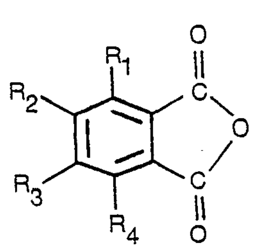

III

+

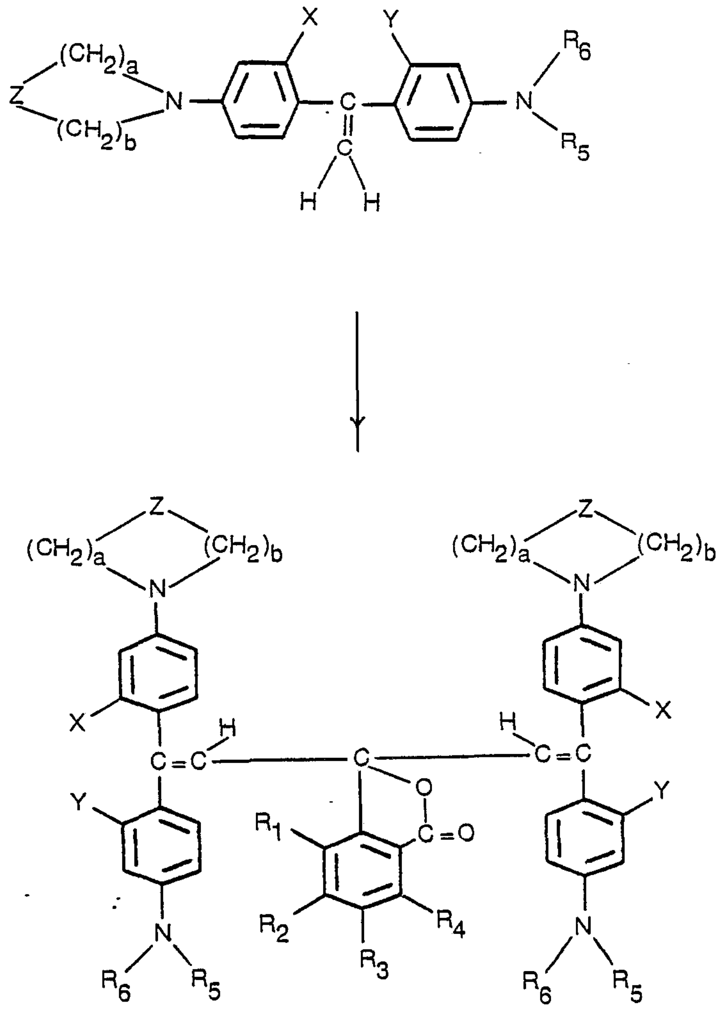

IV

I

The reaction is preferably carried out with the use of a dehydration condensation agent and preferaby at 50 to 200°C for about 30 minutes to several hours.

Phthalic anhdride derivatives of the general formula (III) which are used in the present invention include the following compounds: phthalic anhydride, 3(or 4)-chlorophthalic anhydride, 4,5-dichlorophthalic anhydride, 3,4,5,6-tetrachlorophthalic anhydride, 3(or 4)-bromophthalic anhydride, 4,5-dibromophthalic anhydride, 3,4,5,6-tetrabromophthalic anhyrdide, 4-methylphthalic anhydride, 4-methoxyphthalic anhydride, 4-ethoxyphthalic anhydride, 3(or 4)-nitrophthalic anhydride, 3(or 4)-dimethylaminophthalic anhydride, 3(or 4)-diethylaminophthalic anhydride, 4-(N-ethyl-N-benzyl)aminophthalic anhydride, 4-(N-methyl-N-phenyl)aminophthalic anhydride, and 4-pyrrolidinophthalic anhydride.

Ethylene derivatives of the general formula (IV) which may be used in this invention include 1,1-bis(4-pyrrolidinophenyl)ethylene, 1,1-bis(4-piperidinophenyl)ethylene, 1,1-bis(4-morpholinophenyl)ethylene, 1,1-bis(4-hexamethyleneiminophenyl)ethylene, 1,1-bis(2-methyl-4-pyrrolidinophenyl)ethylene, 1,1-bis(2-methyl-4-piperidinophenyl)ethylene, 1,1-bis(2-methyl-4-morpholinophenyl)ethylene, 1,1-bis(2-methyl-4-hexamethyleneiminophenyl)ethylene, 1,1-bis(2-methoxy-4-pyrrolidinophenyl)ethylene, 1,1-bis(2-methoxy-4-piperidinophenyl)ethylene, 1,1-bis(2-methoxy-4-morpholinophenyl)ethylene, 1,1-bis(2-methoxy-4-hexamethyleneiminophenyl)ethylene, 1-(4-pyrrolidinophenyl)-1-(4-dimethylaminophenyl)ethylene, 1-(4-piperidinophenyl)-1-(4-dimethylaminophenyl)ethylene, 1-(4-morpholinophenyl)-1-(4-dimethylaminophenyl)ethylene, 1-(4-hexamethyleneiminophenyl)-1-(4-dimethylaminophenyl)ethylene, 1-(4-pyrrolidinophenyl)-1-(4-N-ethyl-N-benzylaminophenyl)ethylene, 1-(4-piperidinophenyl)-1-(4-N-ethyl-N-benzylaminophenyl)ethylene, 1-(4-morpholinophenyl)-1-(4-N-ethyl-N-benzylaminophenyl)ethylene, 1-(4-hexamethyleneiminophenyl)-1-(4-N-ethyl-N-benzylaminophenyl)ethylene, 1-(4-pyrrolidinophenyl)-1-(4-N-methyl-N-p-tolylaminophenyl)ethylene, 1-(4-piperidinophenyl)-1-(4-N-methyl-N-p-tolylaminophenyl)-ethylene, 1-(4-morpholinophenyl)-1-(4-N-methyl-N-p-tolylaminophenyl)ethylene, 1-(4-hexamethylene-iminophenyl)-1-(4-N-methyl-N-p-tolylaminophenyl)ethylene, 1-(4-pyrrolidinophenyl)-1-(2-methyl-4-diethylaminophenyl)ethylene, and 1-(4-pyrrolidinophenyl)-1-(2-methoxy-4-diethylaminophenyl)ethylene.

The dehydration condensation agents used for carrying out the reaction between phthalic anhydride derivatives of the general formula (III) and ethylene derivatives of the general formula (IV) may preferably function as solvents. Such dehydration condensation agents include lower fatty acid anhydrides e.g., acetic anhydride and propionic anhydride, and inorganic acids e.g., phosphorus oxychloride, phosphorous trichloride, sulfuric acid and polyphosphoric acid. Various Friedel-Crafts type catalysts may also be used as dehydration condensation agents. These dehydration condensation agents may be used either alone or in combination.

The phthalide derivatives according to the invention are substantially colourless chromogenic compounds which can develop a colour upon contact with electron accepting acidic materials (hereinafter referred to as "acceptors"). They are especially advantageous in that they have good light resistance and show a good absorption for infrared rays. Accordingly, they find their usefulness in use for the production of various kinds of record materials.

The above mentioned phthalide derivatives may be used either solely or in combination or, when desired, together with any of the following basic dye compounds: triarylmethanelactone compounds such as 3,3-bis(p-dimethylaminophenyl)-6-dimethylaminophthalide, 3-(p-dibenzylaminophenyl)-3-(1,2-dimethylindole-3-yl)-7-azaphthalide, 3-(4-diethylamino-2-ethoxyphenyl)-3-(1-ethyl-2-methylindole-3-yl)-7-azaphthalide and 3,3-bis(1-ethyl-2-methylindole-3-yl)phthalide; fluoran compounds such as 3-diethylamino-6-methylfluoran, 3-diethylamino-6-methyl-7-chlorofluoran, 3-(N-ethyl-N-p-tolylamino)-7-methylfluoran, 3-diethylamino-6-methyl-7-anilinofluoran, 3-(N-cyclohexyl-N-methylamino)-6-methyl-7-anilinofluoran, 3-(N-ethyl-N-p-tolylamino)-6-methyl-7-anilinofluoran, and 3-diethylamino-7-o-chloroanilinofluoran; spiropyran compunds such as di-β-naphthospiropyran and 3-methyl-di-β-naphthospiropyran; diphenylmethane compounds such as 4,4'-bis-dimethylaminobenzhydrylbenzyl ether, and 4,4'-bis-dimethylaminophenyl-p-toluenesulfinic acid ester; azine compounds such as 3,7-bis(dimethylamino)-10-benzoyl-phenothiazine and 3,7-bis (diethylamino)-10-benzoylphenoxazine; triarylmethane compounds such as N-butyl-3-[bis{4-(N-methyl-anilino)phenyl}methyl]carbazole.

The acceptors used are selected according to the kinds of recording materials. The materials which are preferably used as acceptors for pressure sensitive record materials, heat sensitive record materials, electrothermal record material, ultrasonic record materials, electrostatic record materials, typewriter ribbons, ball-point pen ink and crayon are those which function as Brønsted or Lewis acid. Among them are included: inorganic acceptors such as acid clay, activated clay, attapulgite, bentonite, colloidal silica, aluminium silicate, magnesium silicate, zinc silicate, tin silicate, calcined kaolin and talc; organic acceptors such as aliphatic carboxylic acids, e.g., oxalic acid, maleic acid, tartaric acid, citric acid, succinic acid and stearic acid, aromatic carboxylic acids, e.g. benzoic acid, p-tert-butylbenzoic acid, phthalic acid, gallic acid, salicylic acid, 3-isopropylsalicylic acid, 3-phenylsalicylic acid, 3-cyclohexylsalicylic acid, 3,5-di-tert-butylsalicylic acid, 3-methyl-5-benzylsalicylic acid, 3-phenyl-5-(α,α-dimethylbenzyl)-salicylic acid, 3,5-di(α-methylbenzyl)salicylic acid and 2-hydroxy-1-benzyl-3-naphthoic acid, phenolic compounds, e.g., 4,4'-isopropylidenediphenol, 4,4'-isopropylidenebis(2-chlorophenol), 4,4'-isopropylidenebis(2,6-dibromophenol, 4,4'-isopropylidenebis(2,6-dichlorophenol), 4,4'-isopropylidenebis(2-methylphenol), 4,4'-isopropylidenebis(2,6-dimethylphenol), 4,4'-isopropylidenebis(2-tert-butylphenol), 4,4'-sec-butylidenebisphenol, 4,4'-cyclohexylidenebisphenol, 4,4'-cyclohexylidenebis (2-methylphenol), 4-tert-butyphenol, 4-phenylphenol, 4-hydroxydiphenoxide, α-naphthol, β-naphthol, methyl-4-hydroxybenzoate, benzyl-4-hydroxybenzoate, 2,2'-thiobis(4,6-dichlorophenol), 4-tert-octylcatechol, 2,2'-methylenebis(4-chlorophenol), 2,2'-methylenebis(4-methyl-6-tert-butyphenol) and 2,2'-dihydroxydiphenyl, phenol resins, e.g., p-phenylphenol-formaldehyde resin and p-butylphenol-acetylene resin; salts of the above organic acceptors with polyvalent metals such as zinc, magnesium, aluminium, calcium, titanium, manganese, tin and nickel; and inorganic acid such as hydrogen halide, e.g., hydrogen chloride, hydrogen bromide and hydrogen iodide, boric acid, silicic acid, phosphoric acid, sulfuric acid, nitric acid, perchloric acid and halides of aluminium, zinc, nickel, tin, titanium and boron.

In case of electron beam record materials or photosensitive record materials, compounds which can produce by electron beam or light radiation hydrogen halides, such as hydrogen chloride, hydrogen bromide and hydrogen iodide, carboxylic acids, sulfonic acids or phenols are preferably used as acceptor materials. Among these compounds, there are included organic halogen compounds, such as carbon tetrabromide, α,α,α-tribromoacetophenone, hexachloroethane, iodoform, 2-tribromomethylpyridine, trichloromethyl sulfonylbenzene, o-quinonediazido compounds, phenol esters of carboxylic acid or sulfonic acid which can cause Fries rearrangement.

Some embodiments of the utilization of the phthalide derivatives according to the invention for various kinds of record materials are described hereinbelow:

The phthalide derivatives can be utilized for various kinds of pressure sensitive record materials, e.g., those disclosed in US—A—2,505,470, 2,505,471, 2,505,489, 2,548,366, 2,712,507, 2,730,456, 2,730,457, 3,418,250, 3,924,027 and 4,010,038.

A typical method for the production of a pressure sensitive record material utilizing the phthalide derivatives according to the invention is as follows:

At least one of the phthalide derivatives according to the invention is dissolved in a solvent to form a solution which may include synthetic oil such as alkylated naphthalene, alkylated diphenyl, alkylated

diphenylmethane and alkylated terphenyl, vegetable oil such as cotton seed oil and caster oil, animal oil and mineral oil or mixtures of the foregoing. The solution may additionally include basic colourless chromogenic materials such as triarylmethane lactones, spirpyrans, fluorans, diphenylmethanes and Leucomethylene Blue. The solution of the phthalide derivative may be dispersed in a binder to form a coating composition. The solution may be enclosed in microcapsules through the utilization of the coacervation technique, the interfacial polymerization technique, the in-situ polymerization technique or any other method for making oil droplet-containing microcapsules and the microcapsules thus prepared are dispersed in a binder to form a coating composition.

Any one of the compositions thus prepared is applied to a base sheet such as a paper sheet, plastic sheet, resin coated paper sheet, to obtain a pressure sensitive record material. In case where the pressure sensitive copying system consists of a top sheet, a bottom sheet and, if necessary, at least one middle sheet, the pressure sensitive record material according to the invention is used as the top sheet and the middle sheet. The pressure sensitive record material according to the invention also be utilized in the "self contained" system in which both the colourless chromogenic material and the acceptor are disposed on one surface of the same sheet. The pressure sensitive record material utilizing the phthalide derivative according to the invention can produce clear colour images having a good light resistance and showing a good absorption for infrared rays which enables a certain reading by an optical reading machine.

The phthalide derivatives according to the invention are also useful for production of various kinds of heat sensitive record materials, e.g., as disclosed in Japanese Patent Publications Nos. 3,680 or 1969, 27,880 of 1969, 14,039 of 1970, 43,830 of 1973, 69 of 1974, 70 of 1974 and 20,142 of 1977. Most typically, heat sensitive record materials may be produced by coating a coating composition including a binder, fine particles of the phthalide derivative according to the invention and the acceptor on a base sheet such as paper sheet, plastic film, synthetic paper sheet, woven fabric sheet or mold.

The amount of the acceptor in the recording layer may be within the range of 1 to 50 parts by weight, preferably within the range of 4 to 10 parts by weight, per one part by weight of the phthalide derivative used. The coating composition may include inorganic metal compounds such as oxides, hydroxides and carbonates of polyvalent metals and/or inorganic pigments in an amount of 0.1 to 5 parts by weight, preferably, 0.2 to 2 parts by weight, per one part by weight of the amount of the acceptor. The recording layer may also include dispersing agents, ultra-violet ray absorbing agents, heat fusible materials, antifoaming agent, fluorescent dye, colouring dyes and other adding materials. The phthalide derivative and the acceptor may be applied to a bse sheet either in the form of a single coating composition or in the form of two separate coating compositions which may be applied one by one. Application of the phthalide derivative and acceptor to a base sheet may also be carried out by impregnation or by sizing. The amount of the coating composition including the phthalide derivative and the acceptor may preferably be within the range of 2 to 12 g/cm$^2$. Among the useful binder materials there may be included starches, celluloses, proteins, gum arabic, polyvinyl alcohol, salts of styrenemaleic anhydride copolymer, styrene-butadiene copolymer emulsions, salts of vionyl acetate-maleic anhydride copolymer and salts of polyacrylic acid.

The electrothermal record materials may be produced according to any known methods such as those disclosed in Japanese Laid-Open Patent Publications Nos. 11,334 of 1974 and 48,930 of 1975. Usually, the record material of this type may be produced, either by coating on a base sheet such as a paper sheet, a coating composition consisting of a dispersion of an electro-conductive material, a basic dye material essentially comprising the phthalide derivative according to the invention, an acceptor and a binder, or by coating an electro-conductive material on a basic sheet to form an electro-conductive layer thereon and further coating on the electro-conductive layer another coating composition consisting of a dispersion of the phthalide derivative according to the invention, an acceptor and a binder. In case where each of the phthalide derivative and the acceptor used is not fusible within the temperature range of 70 to 120°C, an appropriate heat fusible material may be added for controlling the heat sensitivity.

the photosensitive record materials in which the phthalide derivatives according to the invention are utilized may be produced in a similar manner to any of those disclosed in Japanese Patent Publications Nos. 24,188 of 1963, 10,550 of 1970, 13,258 of 1970, 204 of 1974, 6,212 of 1974 and 28,449 of 1974 and Japanese Laid-Open Patent Publications Nos. 31,615 of 1972, 32,532 of 1973, 9,227 of 1974, 135,617 of 1974, 80,120 of 1975, 87,317 of 1975 and 126,228 of 1975.

The invention is also applicable to other recording systems, such as, the ultrasonic record material, e.g., as disclosed in FR—A—2,120,922, the electron beam recording system, e.g., as disclosed in BE—A—7,959,986, the electrostatic record material, e.g., as disclosed in Japanese Patent Publication No. 3,932 of 1974, the photosensitive printing material, e.g., as disclosed in Japanese Laid-Open Patent Publication No. 12,104 of 1973, the seal stamping material, e.g., as disclosed in Japanese Patent Publication No. 10,766 of 1972, type ribbons as disclosed in Japanese Laid-Open Patent Publication No. 3,713 of 1974, ball-point pen ink as disclosed in Japanese Laid-Open Patent Publicatioan No. 83,924 of 1973 and crayon as disclosed in US—A—3,769,045, by merely using the phthalide derivatives instead of the conventional basic colourless chromogenic materials.

## Examples

The following examples serve to illustrate the invention in more detail. Unless otherwise indicated, parts and % signify parts by weight and % by weight, respectively.

Example 1

3.3 g of phthalic anhydride and 12.8 g of 1,1-bis(4-pyrrolidinophenyl)ethylene were added to 25 g of acetic anhydride and the mixture was reacted at 80°C for one hour. After the termination of reaction, the product was poured into water and then neutralized with an aqueous solution of ammonium to decompose acetic anhydride. The resultant precipitate was separated by filtration, dried and recrystallized from ethanol to obtain 6.7 g of 3,3-bis{1,1-bis(4-pyrrolidinophenyl)ethylene-2-yl}phthalide in the form of light green crystals having a melting point of 198°—201°C. The phthalide derivative became blue green upon contact with silica gel.

Examples 2 to 11

Example 1 was repeated except that phthalic anhydride derivatives shown in the Table below were used instead of phthalic anhydride and ethylene derivatives shown in the Table were used instead of 1,1-bis(4-pyrrolidinophenyl)ethylene to obtain the corresponding phthalide derivatives. The colors formed upon contact with silica gel are also shown in the Table.

TABLE

| Example | phthalic anhydride derivatives | ethylene derivatives | phthalide derivatives | colour |
|---|---|---|---|---|
| 2 | 4-nitrophthalic anhydride | 1,1-bis(4-pyrrolidino-phenyl)ethylene | 3,3-bis{1,1-bis(4-pyrrolidino-phenyl)ethylene-2-yl}-5-nitro-phthalide<br>3,3-bis{1,1-bis(4-pyrrolidino-phenyl)ethylene-2-yl}-6-nitro-phthalide | green |
| 3 | 4-ethoxyphthalic anhydride | " | 3,3-bis{1,1-bis(4-pyrrolidino-phenyl)ethylene-2-yl}-5-ethoxy-phthalide<br>3,3-bis{1,1-bis(4-pyrrolidino-phenyl)ethylene-2-yl}-6-ethoxy-phthalide | blue green |
| 4 | 4-methylphthalic anhydride | " | 3,3-bis{1,1-bis(4-pyrrolidino-phenyl)ethylene-2-yl}-5-methyl-phthalide<br>3,3-bis{1,1-bis(4-pyrrolidino-phenyl)ethylene-2-yl}-6-methyl-phthalide | blue green |
| 5 | 4-pyrrolidino-phthalic anhydride | " | 3,3-bis{1,1-bis(4-pyrrolidino-phenyl)ethylene-2-yl}-5-pyrrolidinophthalide<br>3,3-bis{1,1-bis(4-pyrrolidino-phenyl)ethylene-2-yl}-6-pyrrolidinophthalide | green blue |
| 6 | 4,5-dichlorophthalic anhydride | " | 3,3-bis{1,1-bis(4-pyrrolidino-phenyl)ethylene-2-yl}-5,6-dichlorophthalide | green |
| 7 | phthalic anhydride | 1,1-bis(4-piperidino-phenyl)ethylene | 3,3-bis{1,1-bis(4-piperidino-phenyl)ethylene-2-yl} phthalide | blue green |

TABLE (continued)

| Example | phthalic anhydride derivatives | ethylene derivatives | phthalide derivatives | colour |
|---|---|---|---|---|
| 8 | 4-dimethylamino-phthalic anhydride | 1,1-bis(4-piperidino-phenyl)ethylene | 3,3-bis{1,1-bis(4-piperidinophenyl)ethylene-2-yl}-5-dimethyl-aminophthalide<br>3,3-bis{1,1-bis(4-piperidinophenyl)ethylene-2-yl}-6-dimethyl-aminophthalide | green blue |
| 9 | 3,4,5,6-tetra-chlorophthalic anhydride | 1,1-bis(2-methyl-4-pyrrolidinophenyl)-ethylene | 3,3-bis{1,1-bis(2-methyl-4-pyrrolidinophenyl)ethylene-2-yl}-4,5,6,7-tetrachloro-phthalide | green |
| 10 | " | 1,1-bis(2-methoxy-4-pyrrolidino-phenyl)ethylene | 3,3-bis{1,1-bis(2-methoxy-4-pyrrolidinophenyl)ethylene-2-yl}-4,5,6,7-tetrachloro-phthalide | green |

Example 11

A heat-sensitive record material was prepared by the following method with the use of 3,3-bis[1,1-bis(4-pyrrolidinophenyl)ethylene-2-yl]phthalide obtained in Example 1.

(1) Preparation of A liquid:

The following composition was passed through a sand mill

| | |
|---|---|
| phthalimide derivative obtained in Example 1 | 5 parts |
| stearic acid amide | 1 part |
| 2% aqueous solution of hydroxyethylcellulose | 25 parts |

Pulverization was continued until an average particle size of 2 microns.

(2) Preparation of B liquid:

The following composition was passed through a sand mill

| | |
|---|---|
| 4,4'-isopropylidenediphenol | 50 parts |
| stearic acid amide | 10 parts |
| 2% aqueous solution of hydroxyethylcellulose | 250 parts |

Pulverization was continued until an average particle size of 2 microns.

(3) Making a heat-sensitive record material:

The following composition was mixed to prepare a coating composition.

| | |
|---|---|
| A liquid | 62 parts |
| B liquid | 31 parts |
| ultrafinely divided particles of silicic anhydride ("Syloid 244" manufactured by Fuji-Davidson Chemical) | 25 parts |
| 20% aqueous solution of a salt of styrene-maleic anhydride copolymer | 175 parts |
| zinc stearate | 5 parts |
| water | 100 parts |

The coating composition was coated on a base sheet of 50 g/m$^2$ in the weight of an amount of 6 g/m$^2$ on dry basis to obtain a heat-sensitive record material.

The heat-sensitive record material was pressed with a pressure of 4 kg/cm$^2$ for 5 seconds on a plate heated at 125°C to develop blue green images. The colour images were superior in light resistance. The colour change and discolouration when exposed to sunlight were not substantially appreciated. The light absorption spectrum of the colour images had a broad strong absorption at 600—900 nm.

Example 12

A pressure-sensitive record material was prepared by the following method with the use of the mixture of 3,3-bis[1,1-bis(4-pyrrolidinophenyl)ethylene-2-yl-5-pyrrolidinophthalide and [3,3-bis[1,1-bis(4-pyrrolidinophenyl)ethylene-2-yl]-6-pyrrolidinophthalide obtained in Example 5.

3 parts of the above phthalide derivative were dissolved in 100 parts of isopropylated naphthalene. The resultant solution was dispersed in 350 parts of warm water (50°) containing 25 parts of pigskin-gelatin having an isoelectric point of 8 and 25 parts of gum arabic dissolved in it to obtain an emulsion. 1000 parts of warm water were added to the emulsion. The mixture was adjusted to pH 4 with acetic acid and cooled at 10°C. 10 parts of 25% aqueous solution of glutaraldehyde was added to it to solidify capsules. The capsule-containing coating composition was coated on one surface of a base sheet of 45 g/m$^2$ in the weight of 5 g/m$^2$ on dry basis and an acceptor coating composition comprising 20 parts of zinc 3,5-bis(α-methylbenzyl)salicylate, 80 parts of kaolin and 30 parts of styrene-butadiene copolymer emuylsion (solid content: 50) dispersed in 200 parts of water was coated on another surface of the base sheet in the weight of 5 g/m$^2$ on dry basis to obtain a pressure-sensitive record material (middle sheet).

Several of the pressure-sensitive record material, piled in a manner such that the capsule coated layer was close to the acceptor coated layer, were pressed with driving a pen to develop blue green colour

images on the acceptor coated surface. The colour images were stable to water and alcohol and when exposed to sunlight the color change and discolouration were not appreciated. The light absorption spectrum had a broad strong absorption at 590—900 nm.

Example 13

Example 12 was repeated except that 3 parts of the mixture of 3,3-bis[1,1-bis(4-piperidinophenyl)ethylene-2-yl]-5-dimethylaminophthalide and 3,3-bis[1,1-bis(4-piperidinophenyl)-ethylene-2-yl]-6-dimethylaminophthalide obtained in Example 8 to obtain a pressure-sensitive record material and to develop colour images. The colour images were superior in light resistance and the light absorption spectrum of them had a broad strong absorption at 595—900 nm.

**Claims**

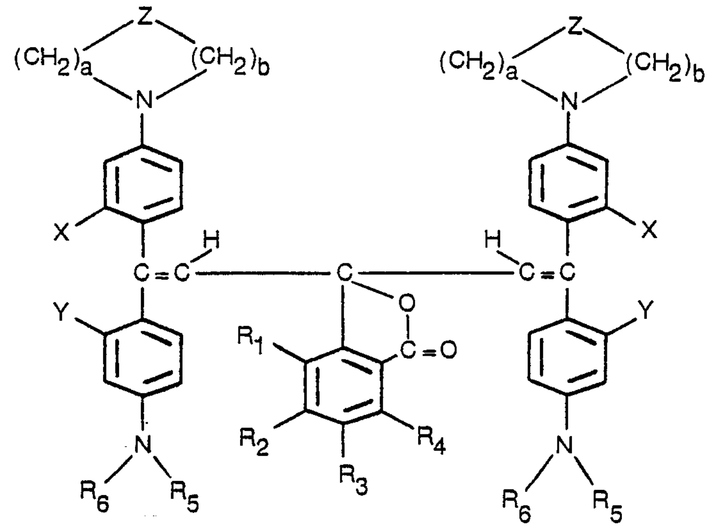

I

wherein:

each of $R_1$, $R_2$, $R_3$ and $R_4$ is hydrogen, halogen, $C_{1-4}$alkyl, $C_{1-4}$alkoxy, nitro, $C_{1-4}$alkyl substituted amino or pyrrolidino;

each of $R_6$ and $R_5$ adjacent a common nitrogen atom is hydrogen, $C_{1-4}$alkyl, benzyl, phenyl or $C_{1-4}$alkyl substituted phenyl or $R_6$ and $R_5$ together with the adjacent nitrogen atom form a pyrrolidino, piperidino, hexamethylenimino or morpholino ring, both $R_6$' being the same as each other and both $R_5$'s being the same as each other;

X is hydrogen, $C_{1-4}$alkyl or $C_{1-4}$alkoxy, both X's being the same as each other;

Y is hydrogen, $C_{1-4}$alkyl or $C_{1-4}$alkoxy, both Y's being the same as each other;

each Z is oxygen or methylene, both Z's being the same as each other;

each of a and b is an integer of from 1 to 3, both a's being the same as each other and both b's being the same as each other; and

in each N-heterocyclic ring $a + b \geq 3$;

provided that when all of $R_1$, $R_2$, $R_3$ and $R_4$ are chlorine or bromine X and/or Y is $C_{1-4}$alkyl or $C_{1-4}$alkoxy.

2. A phthalide derivative according to claim 1, in which said phthalide derivative has the general formula (II)

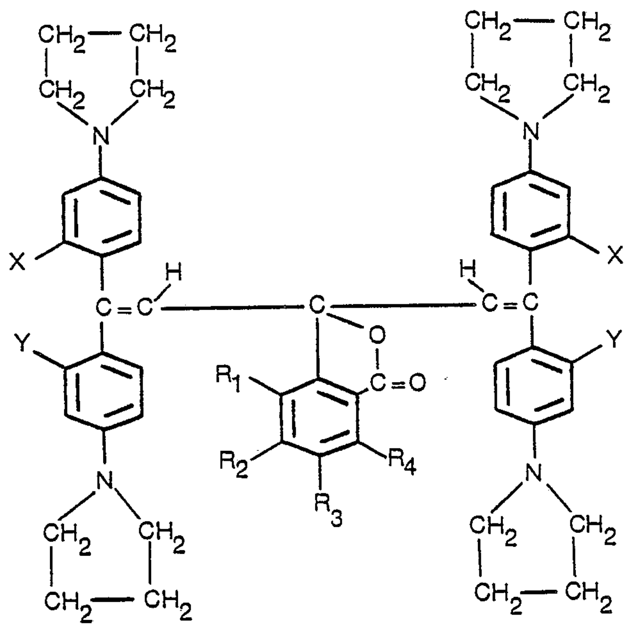

II

wherein $R_1$, $R_2$, $R_3$, $R_4$, X and Y are as hereinbefore defined.

3. A process for preparing a phthalide derivative as defined in Claim 1, which comprises reacting together a phthalic anhydride derivative having the general formula (III)

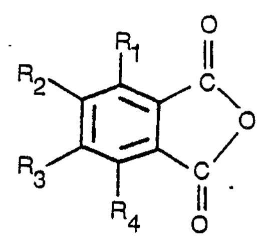

III

and an ethylene derivative having the general formula (IV)

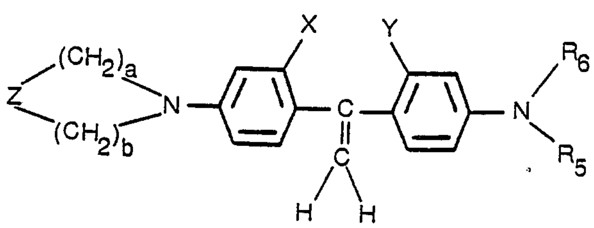

IV

wherein $R_1$, $R_2$, $R_3$, $R_4$, $R_5$, $R_6$, X, Y, Z, a and b are as hereinbefore defined.

4. A recording system comprising a colourless chromogenic material and an electron accepting acidic reactant material and which utilizes a colour forming reaction between said materials, characterized in that said colourless chromogenic material comprises a phthalide derivatives as defined in either of claims 1 and 2.

5. A recording system according to claim 4, in which said recording system is a pressure-sensitive recording system.

6. A recording system according to claim 4, in which said recording system is a heat-sensitive recording system.

**Patentansprüche**

1. Phthalid-Derivat der allgemeinen Formel (I)

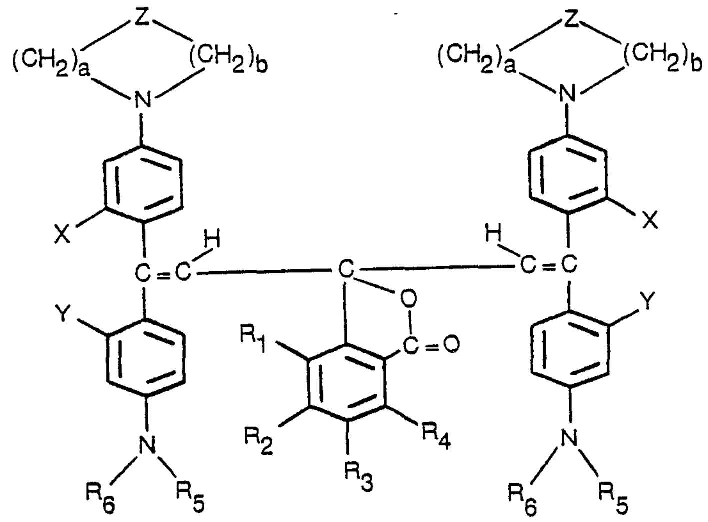

I

worin bedeuten

$R_1$, $R_2$, $R_3$ und $R_4$ jeweils Wasserstoff, Halogen, $C_{1-4}$-Alkyl, $C_{1-4}$-Alkoxy, Nitro, $C_{1-4}$-alkylsubstituiertes Amin oder Pyrrolidin;

$R_5$ und $R_6$ jeweils, einem Stickstoffatom benachbart, Wasserstoff, $C_{1-4}$-Alkyl, Benzyl, Phenyl oder $C_{1-4}$-alkylsubstituiertes Phenyl, oder $R_5$ und $R_6$ zusammen mit dem benachbarten Stickstoffatom einen Pyrrolidin-, Piperidin-, Hexamethylenimin- oder Morpholin-Ring bildend, wobei die beiden $R_5$ jeweils gleich sind und die beiden $R_6$ jeweils gleich sind;

X Wasserstoff, $C_{1-4}$-Alkyl oder $C_{1-4}$-Alkoxy, wobei die beiden X jeweils gleich sind;

Y Wasserstoff, $C_{1-4}$-Alkyl oder $C_{1-4}$-Alkoxy, wobei die beiden Y jeweils gleich sind;

Z Sauerstoff oder Methylen, wobei die beiden Z jeweils gleich sind;

a und b jeweils eine Zahl von 1 bis 3, wobei die beiden a jeweils gleich sind und die beiden b jeweils gleich sind, und in jedem heterocyclischen Ring $a + b \geq 3$ ist;

vorausgesetzt, daß — faalls alle $R_1$, $R_2$, $R_3$ und $R_4$ Chlor oder Brom sind —X und/oder Y $C_1$—$C_4$-Alkyl oder $C_{1-4}$-Alkoxy sind.

2. Phthalid-Derivat nach Anspruch 1, worin das Phthalid-Derivat die allgemeine Formel (II) hat

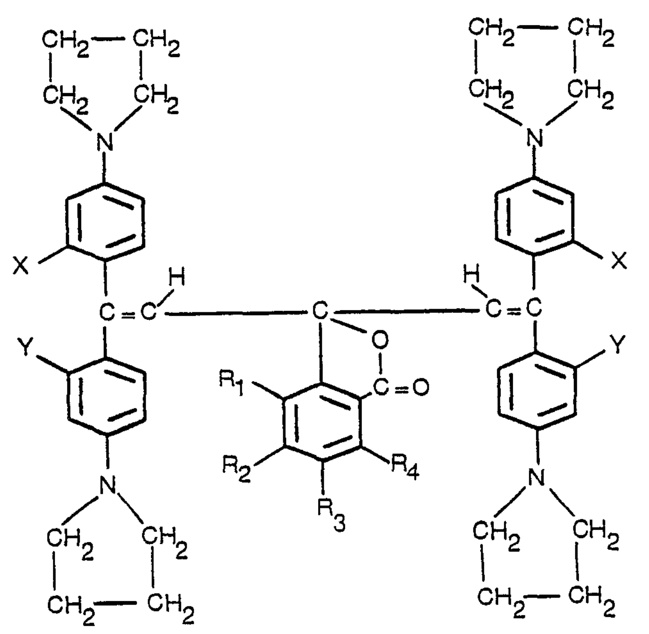

II

worin $R_1$, $R_2$, $R_3$, $R_4$, X und Y die oben angegebene Bedeutung aufweisen.

3. Verfahren zur Herstellung eines Phthalid-Derivates nach Anspruch 1 durch gemeinsame Umsetzung eines Phthalsäureanhydrid-Derivates der allgemeinen Formel (III)

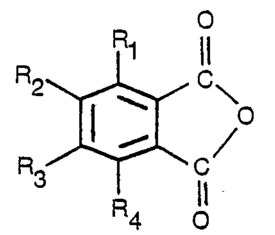

III

und eines Ethylenderivates der allgemeinen Formel (IV)

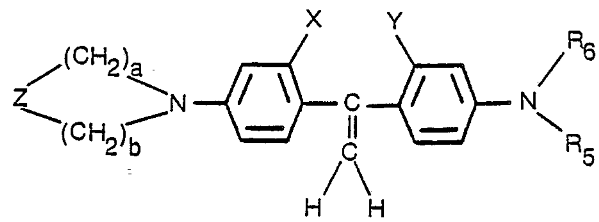

IV

worin $R_1$, $R_2$, $R_3$, $R_4$, $R_5$, $R_6$, X, Y, Z, a und b wie vorstehend definiert sind.

4. Aufzeichnungssystem umfassend ein farbloses chromogenes Material und ein elektronenaufnehmendes saures Reaktan tenmaterial, das auf der farbbildenden Reaktion zwischen diesen Materialien basiert, dadurch gekennzeichnet, daß das farblose chromogene Material ein Phthalid-Derivat nach Anspruch 1 oder Anspruch 2 enthält oder daraus besteht.

5. Aufzeichnungssystem nach Anspruch 4, wobei das Aufzeichnungssystem ein druckempfindliches Aufzeichnungssystem ist.

6. Aufzeichnungssystem nach Anspruch 4, wobei das Aufzeichnungssystem ein wärmeempfindliches Aufzeichnungssystem ist.

**Revendications**

1. Un dérivé du phtalide ayant la formule générale (I)

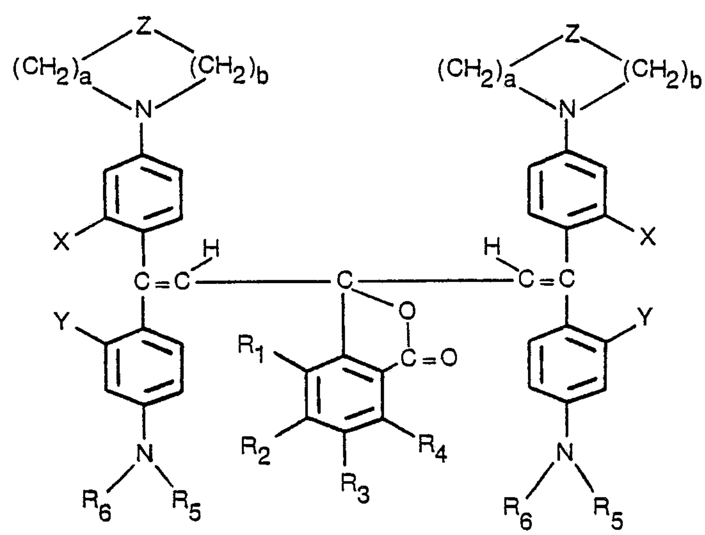

I

dans laquelle:

chacun des groupes $R_1$, $R_2$, $R_3$ et $R_4$ est l'hydrogène, un halogène, un alkyle en $C_{1-4}$, un alkoxy en $C_{1-4}$, un groupe nitro, un groupe amino ou pyrrolidino substitués par un alkyle en $C_{1-4}$;

chacun des groupes $R_6$ et $R_5$ adjacents à un atome d'azote commun est l'hydrogène, un alkyle en $C_{1-4}$, un benzyle, un phényle ou un phényle substitué par un alkyle en $C_{1-4}$ ou bien $R_6$ et $R_5$ forment ensemble avec l'atome d'azote adjacent un cycle pyrrolidino, pipéridino, hexaméthylèneimino ou morpholino, les deux $R_6$ étant identiques l'un à l'autre et les deux $R_5$ étant identiques l'un à l'autre;

X est l'hydrogène, un alkyle en $C_{1-4}$ ou un alkoxy en $C_{1-4}$, les deux X étant identiques l'un à l'autre.
Y est l'hydrogène, un alkyle en $C_{1-4}$ ou un alkoxy en $C_{1-4}$, les deux Y étant identiques l'un à l'autre.
chaque Z est l'oxygène ou le méthylène, les deux Z étant identiques l'un à l'autre;
chacun de a et b est un nombre entier de 1 à 3, les deux a étant identiques l'un à l'autre et les deux b étant identiques l'un à l'autre; et
dans chaque N-hétérocycle a + b ≥ 3;
pourvu que lorsque tous les $R_1$, $R_2$, $R_3$ et $R_4$ sont du chlore ou du brome, X et/ou Y est un alkyle en $C_{1-4}$ ou un alkoxy en $C_{1-4}$.

2. Un dérivé du phtalide selon la revendication 1, dans lequel ledit dérivé du phtalide a la formule générale (II)

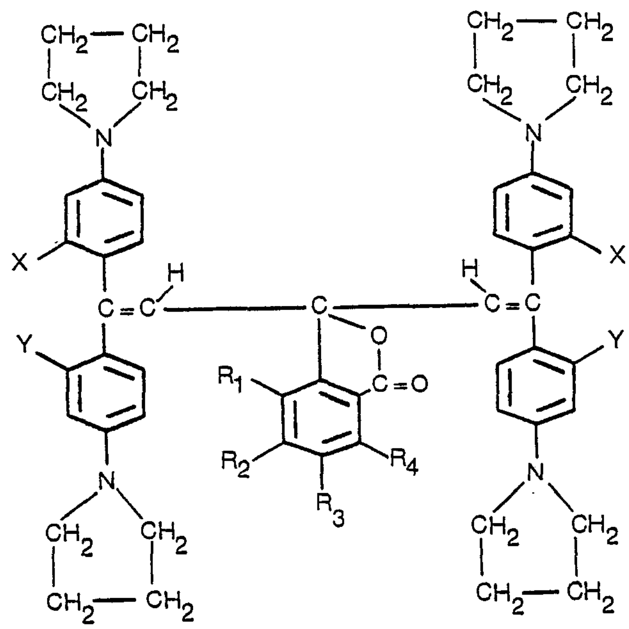

II

dans laquelle $R_1$, $R_2$, $R_3$, $R_4$, X et Y sont tels que définis ci-dessus.

3. Un procédé pour préparer un dérivé du phtalide selon la revendication 1, qui consiste à faire réagir ensemble un dérivé de l'anhydride phtalique ayant la formule générale (III)

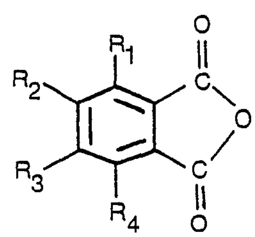

III

et un dérivé de l'éthylène ayant la formule générale (IV),

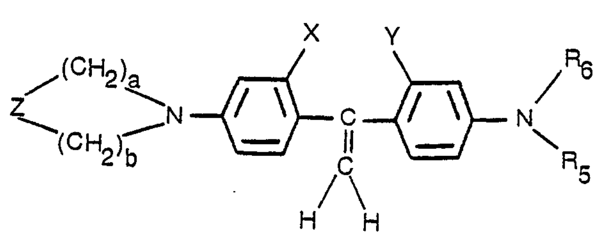

IV

dans laquelle $R_1$, $R_2$, $R_3$, $R_4$, $R_5$, $R_6$, X, Y, Z, a et b sont tels que définis ci-dessus.

4. Un système d'enregistrement comprenant un matériau chromogène incolore et un matériau réagissant acide accepteur d'electron et qui met en oeuvre une réaction formant couleur entre lesdits matériaux caractérisé en ce que ledit matériau chromogène incolore comprend un dérivé du phtalide tel

que défini dans l'une quelconque des revendications 1 et 2.

5. Un système d'enregistrement selon la revendication 4, dans lequel ledit système d'enregistrement est un système d'enregistrement sensible à la pression.

6. Un système d'enregistrement selon la revendication 4, dans lequel ledit système d'enregistrement est un système d'enregistrement sensible à la chaleur.